# FASCICULE DE BREVET EUROPEEN

(11) **EP 1 720 440 B1**
(45) Date de publication et mention de la délivrance du brevet: **04.05.2011**
(21) Numéro de dépôt: 05706541.9
(22) Date de dépôt: 02.03.2005
(51) Int. Cl.: A61B 1/015, A61B 1/12

(54) **SYSTEME D'ENDOSCOPIE ET CONNECTEUR A DETECTEUR DE PRESSION DESTINE A UN TEL SYSTEME**
ENDOSKOPIE-SYSTEM UND DRUCKÜBERMITTELNDER VERBINDER FÜR DIESES SYSTEM
ENDOSCOPY SYSTEM AND A PRESSURE TRANSMITTING CONNECTOR FOR SAID SYSTEM

(30) Priorité: 04.03.2004 FR 0402238
(43) Date de publication de la demande: 15.11.2006
(73) Titulaire: Future Medical System S.A., 1217 Meyrin (CH)
(72) Inventeur: FRANCISCO, André, F-06560 SOPHIA ANTIPOLIS (FR); JANIN, Patrick, F-06000 NICE (FR); PASCAL, Thierry, F-06700 Saint Laurent du Var (FR); DIAS, Armando, F-06300 Nice (FR)
(74) Mandataire: Savoye, Jean-Paul
(86) Numéro de dépôt international: PCT/CH2005/000122
(87) Numéro de publication internationale: WO 2005/084523

(56) Documents cités:
- US-A- 4 132 227
- US-A- 5 044 203
- US-A- 5 643 203
- US-A- 5 810 770

## Description

### Domaine technique

L'invention se rapporte à un système d'endoscopie comprenant plus particulièrement une canule pour loger un endoscope et pour former, entre la canule et l'endoscope, un canal d'irrigation ou d'aspiration et comprenant une bague de raccordement montée autour de la canule et pourvue d'une voie de raccordement pour communiquer avec le canal d'irrigation ou d'aspiration.

### Etat de la technique

Un tel système d'endoscopie est décrit notamment par les documents US 5037386 et US 6086542. Il est utilisé dans l'arthroscopie des articulations et plus particulièrement dans l'arthroscopie du genou. L'endoscope est relié à un écran vidéo pour visualiser l'articulation. Le canal d'irrigation ou d'aspiration permet de créer une circulation d'eau physiologique pour maintenir un milieu optiquement clair devant l'endoscope et pour laver l'articulation. La circulation est assurée par une pompe reliée à un réservoir et débitant dans une tubulure raccordée au canal d'irrigation ou d'aspiration par l'intermédiaire de la bague de raccordement.

La pression de l'eau physiologique dans l'articulation est contrôlée par un détecteur de pression à membrane disposé sur la tubulure du canal d'irrigation ou d'aspiration ou sur une tubulure raccordée par la bague de raccordement à un canal formé dans la canule et dédié à la détection de pression, comme dans le cas des systèmes d'endoscopie décrits par les documents cités précédemment. Ces agencements présentent l'inconvénient de conduire à une détermination erronée de la pression dans certaines conditions d'utilisation, par exemple lorsque la tubulure forme accidentellement un coude entre le détecteur de pression et la bague de raccordement.

Le document US 5643203 divulgue un système d'endoscopie du type rappelé précédemment, dans lequel un connecteur est monté sur la bague de raccordement et comprend une voie de communication avec le canal d'irrigation et un détecteur de pression pour détecter une pression dans la voie de communication. La pression de l'eau physiologique dans l'articulation est extrapolée par une loi à partir de la pression détectée dans la voie de communication.

L'extrapolation de la pression dans l'articulation à partir de la pression détectée dans la voie de communication du connecteur permet de s'affranchir d'un canal dédié dans la canule à la prise de pression. On peut ainsi diminuer le diamètre de la canule, dans le but de réduire le traumatisme lors de son introduction dans l'articulation. Par rapport à une tubulure, le connecteur élimine aussi le risque d'une variation de section de la voie de communication et permet une détection fiable de la pression dans cette voie de communication.

Cependant, la pression détectée reste sujette à une perte de charge artificiellement élevée dans la voie de raccordement de la voie de communication du connecteur au canal d'irrigation de la canule. Il peut s'agir d'un corps étranger présent dans la voie de raccordement de la bague de raccordement et obstruant partiellement la circulation de l'eau physiologique dans le canal d'irrigation.

L'un des buts de l'invention est de palier cet inconvénient afin de conférer un haut degré de sécurité au système d'endoscopie.

### Divulgation de l'invention

A cet effet, l'invention a pour objet un système d'endoscopie comprenant une canule pour loger un endoscope et pour former, entre la canule et l'endoscope, un canal d'irrigation, respectivement d'aspiration, destiné au transport d'un fluide d'irrigation, respectivement d'aspiration, une bague de raccordement montée autour de la canule et pourvue d'une voie de raccordement au canal d'irrigation, respectivement d'aspiration, et un connecteur monté sur la bague de raccordement et comprenant une voie de communication avec la voie de raccordement et un premier détecteur de pression pour détecter une pression dans la voie de communication, caractérisé en ce que la bague de raccordement est pourvue d'une voie de dérivation communiquant avec le canal d'irrigation, respectivement d'aspiration, et en ce que le connecteur comprend une voie borgne communiquant avec la voie de dérivation et un deuxième détecteur de pression pour détecter une pression dans cette voie borgne.

La voie de dérivation de la bague de raccordement communique avec la voie borgne du connecteur pour permettre une deuxième détection de la pression de l'eau physiologique propre circulant dans le canal d'irrigation. Il en va de même lorsque l'on considère l'eau physiologique souillée circulant dans le canal d'aspiration. D'où il résulte que le système d'endoscopie selon l'invention possède une double sécurité pour le contrôle de la pression de l'eau physiologique dans l'articulation.

De préférence, la voie de dérivation est disposée en aval de la voie de raccordement au canal d'irrigation par rapport au transport du fluide en irrigation, respectivement amont de la voie de raccordement au canal d'aspiration par rapport au transport du fluide en aspiration, et en ce qu'un robinet d'irrigation est prévu pour fermer ou ouvrir la voie de raccordement au canal d'irrigation, en amont de la voie de dérivation, respectivement un robinet d'aspiration est prévu pour fermer ou ouvrir la voie de raccordement au canal d'aspiration, en aval de la voie de dérivation. Cet agencement permet avantageusement de toujours contrôler la pression dans l'articulation même lorsque la circulation de l'eau physiologique propre dans le canal d'irrigation est interrompue par la fermeture du robinet d'irrigation. Il en va de même lorsque l'on considère l'eau physiologique souillée circulant dans le canal d'aspiration.

L'invention s'étend à un connecteur destiné à un système d'endoscopie qui comprend une voie de communication et un détecteur de pression pour détecter la pression dans cette voie de communication, caractérisé en ce qu'il comprend une voie borgne et un deuxième détecteur de pression pour détecter la pression dans la voie borgne.

Avantageusement, le connecteur comprend une deuxième voie de communication. De préférence, la voie de dérivation est disposée entre les deux voies de communication. Cet agencement permet de connecter à la bague de raccordement, la voie de communication pour l'irrigation, la voie borgne et la voie de communication pour l'aspiration en une seule opération, tout en permettant une détection de pression dans la voie borgne et l'une ou l'autre voie de communication.

### Brève description des dessins

D'autres avantages de l'invention apparaîtront à la lecture de la description d'un mode de réalisation illustré ci-après par les dessins.

- La figure 1: montre un système d'endoscopie en vue de face.
- La figure 2: montre le système d'endoscopie de la figure 1 en vue de dessus.
- La figure 3: montre le système d'endoscopie de la figure 1 en coupe longitudinale.
- La figure 4: montre le système d'endoscopie de la figure 1 suivant une coupe transversale.
- La figure 5: montre en coupe transversale un premier connecteur destiné à un système d'endoscopie selon l'invention.
- La figure 6: montre en coupe transversale un deuxième connecteur destiné à un système d'endoscopie selon l'invention.

### Mode(s) de réalisation de l'invention

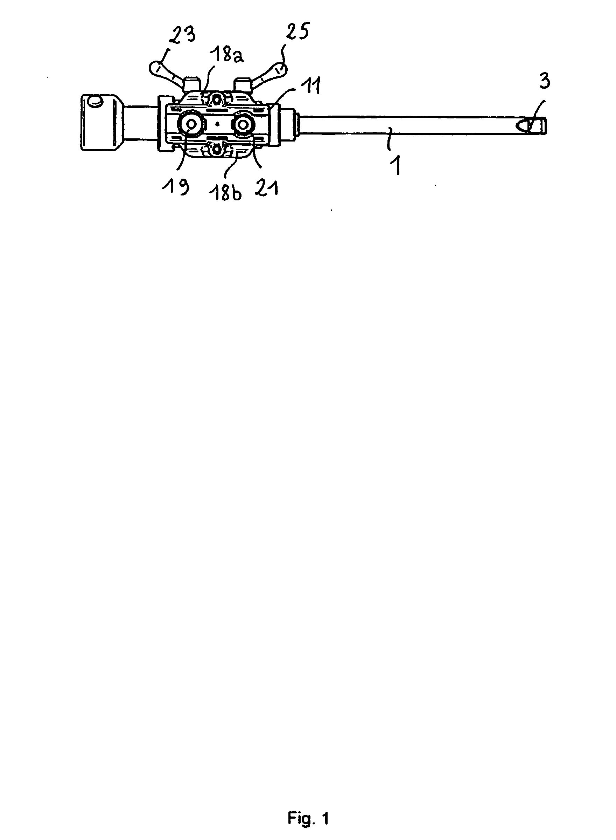

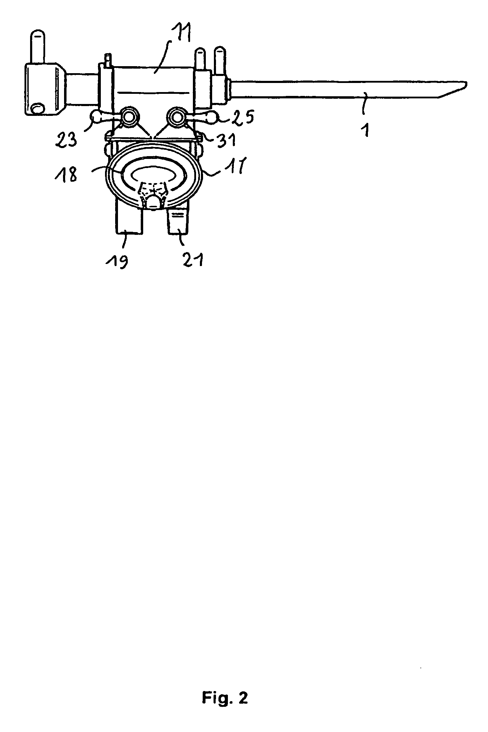

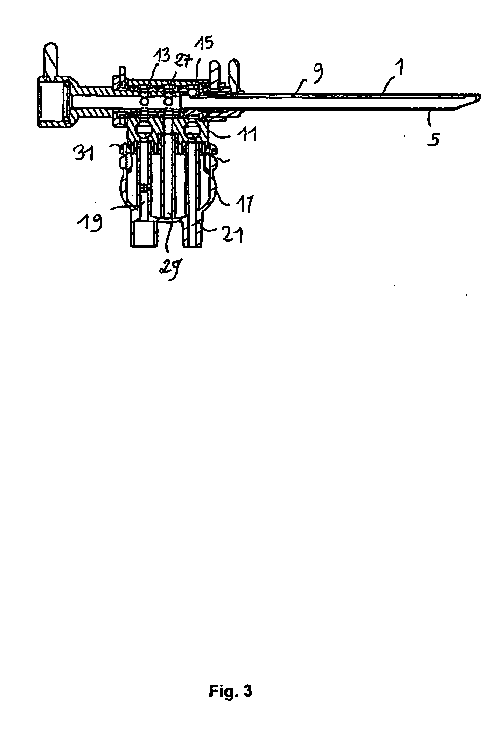

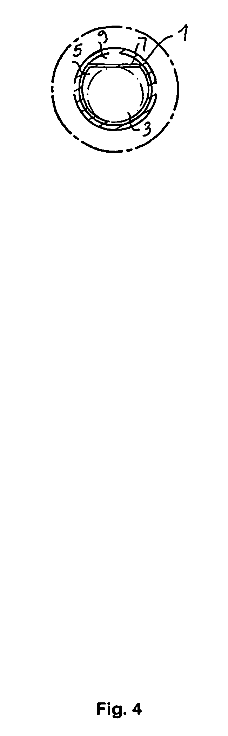

Un système d'endoscopie comprend, figures 1 à 4, une canule 1 pour loger un endoscope 3 et pour former, entre la canule 1 et l'endoscope 3, un canal d'irrigation 5. Dans le mode d'exécution choisi pour illustrer l'invention , le canal d'irrigation 5 est formé entre l'endoscope 3 et un tube 7 interne à la canule 1 et un canal d'aspiration 9 est formé entre le tube interne 7 et la canule 1. Toutefois, l'invention s'applique également à une canule ne comprenant que le canal d'irrigation ou que le canal d'aspiration.

Une bague de raccordement 11 est montée autour de la canule 1 pour communiquer avec le canal d'irrigation 5 et le canal d'aspiration 9. Une première voie de raccordement 13 communique avec le canal d'irrigation 5. Une deuxième voie de raccordement 15 communique avec le canal d'aspiration 9.

Un connecteur 17 est monté sur la bague de raccordement 11. Il comprend une première voie de communication 19 pour communiquer avec la première voie de raccordement 13 au canal d'irrigation 5 et un premier détecteur de pression 18a et comprend une deuxième voie de communication 21 pour communiquer avec la deuxième voie de raccordement 15 au canal d'aspiration 9. Les deux voies de communication 19 et 21 du connecteur 17 sont disposées en regard du premier détecteur de pression 18a pour détecter la pression dans l'une 19 ou l'autre 21 de ces deux voies de communication.

Des tubulures non représentées sont connectées aux voies de communication 19 et 21 du connecteur 17 et reliées à une pompe pour créer une circulation d'eau physiologique propre dans le canal d'irrigation 5 et d'eau physiologique souillée dans le canal d'aspiration 9. De façon connue en soi, la bague de raccordement 11 comprend des robinets 23 et 25 pour ouvrir ou fermer les voies de raccordement 13 et 15 en fonction de la circulation recherchée dans le canal d'irrigation 5 ou dans le canal d'aspiration 9.

Le connecteur 17 est monté sur la bague de raccordement 11 pour permettre au canal d'irrigation 5 et au canal d'aspiration 9 de communiquer avec les voies de communication 13 et 15 du connecteur sans l'intermédiaire d'une tubulure. Par cet agencement, la pression détectée sur l'une ou l'autre voie de communication du connecteur n'est pas sujette à une erreur due à une variation accidentelle de la section de tubulures qui seraient raccordées aux voies de raccordement de la bague de raccordement.

Selon l'invention, la bague de raccordement 11 comprend une voie de dérivation 27 pour communiquer avec la voie de raccordement 13 au canal d'irrigation 5 tandis que le connecteur 17 comprend une voie borgne 29 pour communiquer avec la voie de dérivation 27 et un deuxième détecteur de pression 18b pour détecter une pression dans cette voie borgne. Comme indiqué précédemment, la voie de dérivation 27 de la bague de raccordement communique avec la voie borgne 29 du connecteur pour permettre une deuxième détection de la pression de l'eau physiologique propre circulant dans le canal d'irrigation.

De préférence, la voie de dérivation 27 est disposée, par rapport au transport du fluide en irrigation, en aval de la voie de raccordement 13 au canal d'irrigation 5 et le robinet d'irrigation 23 est prévu pour fermer ou ouvrir cette voie de raccordement 13 en amont de la voie de dérivation 27. De même, la voie de dérivation 27 est disposée, par rapport au transport du fluide en aspiration, en amont de la voie de raccordement 15 au canal d'aspiration 9 et le robinet d'aspiration 25 est prévu pour fermer ou ouvrir cette voie de raccordement 15 en aval de la voie dérivation 27. Cet agencement permet avantageusement de détecter une pression sur une voie, la voie de dérivation, non perturbée par l'ouverture ou la fermeture, même partielle, du robinet 23 de la voie de raccordement 13 au canal d'irrigation 5 ou du robinet 25 de la voie de raccordement 15 au canal d'aspiration 9. De surcroît, la pression dans l'articulation peut être extrapolée par le biais de la voie de dérivation même lorsque la circulation de l'eau physiologique est interrompue dans le canal d'irrigation, respectivement d'aspiration, par la fermeture du robinet d'irrigation 23, respectivement 25.

La deuxième prise de pression via la voie borgne permet de mieux contrôler l'intégrité du système d'endoscopie selon l'invention par comparaison des pressions détectées par les deux détecteurs avec des valeurs attendues. Il est ainsi possible de diagnostiquer une défaillance du robinet d'irrigation 23 par une perte de charge anormale entre la pression détectée par le premier détecteur 18a dans la voie de communication 19 avec le canal d'irrigation 5 et la pression détectée par le deuxième détecteur 18b dans la voie borgne. Il en va de même pour une défaillance du robinet d'aspiration 25. Ces contrôles seront avantageusement effectués par le chirurgien en début d'utilisation du système d'endoscopie.

### Application industrielle

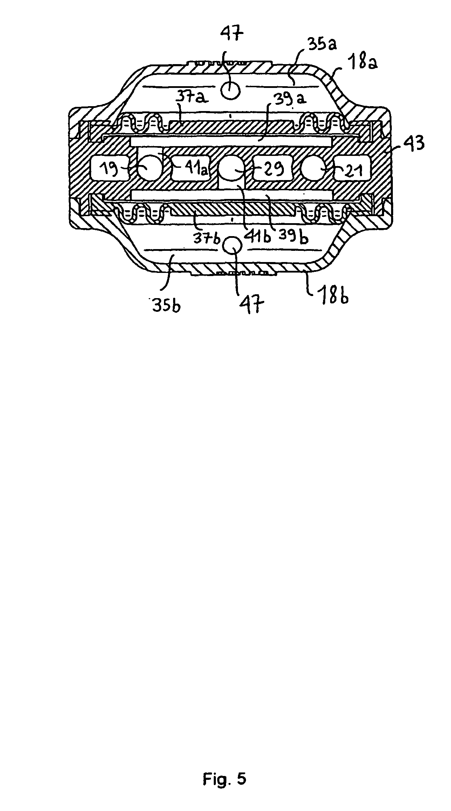

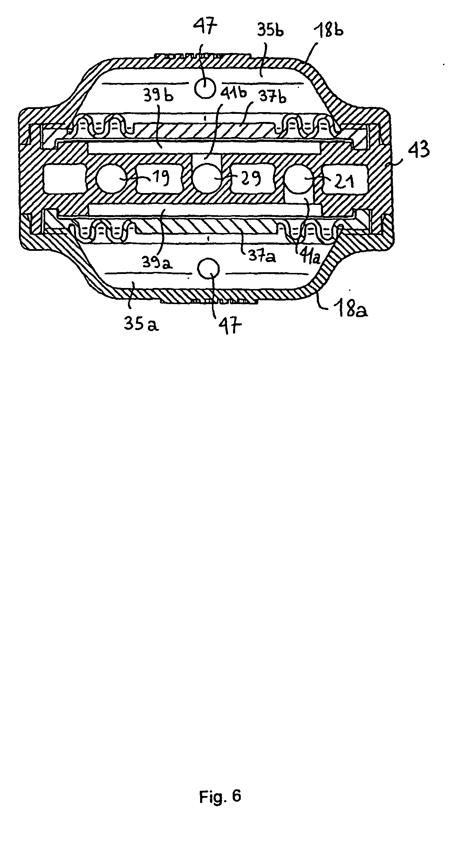

Le connecteur du système d'endoscopie selon l'invention est de préférence, une pièce rigide en matière plastique injectée et intègre deux détecteurs de pression à membrane 18a et 18b. Comme visible sur les figures 5 et 6, le premier 18a et le deuxième 18b détecteurs comprennent chacun une chambre 35a,35b et une membrane 37a,37b prévue pour se déformer et faire varier la pression d'air régnant dans la chambre 35a,35b. De façon plus détaillée, la voie de communication 19,21 ainsi que le conduit 41a,41b et le compartiment à fluide 39a,39b sont formés dans une même pièce rigide 43 sur laquelle la membrane 37a,37b et la chambre de transmission sont rapportées pour fermer la chambre de transmission sur le compartiment à fluide 39a,39b par la membrane 37a,37b. La pièce rigide 43 est pourvue de moyens de fixation 31 à la bague de raccordement 11. De préférence, la pièce rigide 43 est pourvue d'un organe à détromper la connexion sur la bague de raccordement 11. Ces agencements permettent à un chirurgien de connecter le connecteur sur la bague de raccordement d'une façon aisée et sûre.

L'eau physiologique circulant dans la voie de communication 19 avec le canal d'irrigation 5, figure 5, ou dans la voie de communication 21 avec le canal d'aspiration 9, figure 6, pénètre dans le compartiment à fluide 39a du premier détecteur de pression 18a, ouvert sur la voie de communication 13,15 par le conduit 41 a et fermé par la membrane 37a. Cette dernière 37a se déforme en fonction de la pression de l'eau physiologique dans la voie de communication 19 ou 21.

De même, l'eau physiologique circulant dans le canal d'irrigation 5, respectivement d'aspiration 9, pénètre, via la voie de dérivation 27 et la voie borgne 29, dans le compartiment à fluide 39b du deuxième détecteur 18b, ouvert sur la voie borgne 29 par le conduit 41b et fermé par la membrane 37b. Cette dernière se déforme en fonction de la pression de l'eau physiologique dans la voie borgne 29.

Des capillaires non représentés sont branchés sur des prises de branchement 47 des chambres de transmission 37a, 37b pour transmettre les variations de la pression d'air régnant dans les chambres 35a, 35b à des capteurs non représentés et pour déterminer la pression de l'eau physiologique dans le voie de communication 19, 21 et dans la voie borgne 29.

Le système d'endoscopie selon l'invention est particulièrement utile dans l'arthroscopie des articulations, comme celles du genou ou de l'épaule. Comme indiqué précédemment, l'invention s'applique à une canule qui ne comprend pas de canal dédié à la détection de la pression dans l'articulation, pour avantageusement diminuer le diamètre extérieur de la canule pour la rendre moins traumatisante lors de sa mise en place par le chirurgien. Cependant, l'invention s'applique également à un système d'endoscopie où la canule comprend un canal dédié à la pression. La bague de raccordement est de ce fait modifiée pour que la voie de dérivation communique avec le canal dédié à la pression et le connecteur est monté sur la bague de raccordement pour mettre en communication la voie borgne avec la voie de dérivation.

## Revendications

1. Système d'endoscopie comprenant une canule (1) pour loger un endoscope (3) et pour former, entre la canule et l'endoscope, un canal d'irrigation (5), respectivement d'aspiration (9), destiné au transport d'un fluide d'irrigation, respectivement d'aspiration, une bague de raccordement (11) montée autour de la canule (1) et pourvue d'une voie de raccordement (13,15) au canal d'irrigation (5), respectivement d'aspiration (9), et un connecteur (17) monté sur la bague de raccordement (11) et comprenant une voie de communication (19,21) avec la voie de raccordement (13,15) et un premier détecteur de pression (18a) pour détecter une pression dans la voie de communication (19,21), **caractérisé en ce que** la bague de raccordement (11) est pourvue d'une voie de dérivation (27) communiquant avec le canal d'irrigation (5), respectivement d'aspiration (9), et **en ce que** le connecteur (17) comprend une voie borgne (29) communiquant avec la voie de dérivation (27) et un deuxième détecteur de pression (18b) pour détecter une pression dans cette voie borgne (29).

2. Système d'endoscopie selon la revendication 1, **caractérisé en ce que** la voie de dérivation (27) est disposée en aval de la voie de raccordement (13) au canal d'irrigation (5) par rapport au transport du fluide en irrigation, respectivement amont de la voie de raccordement (15) au canal d'aspiration (9) par rapport au transport du fluide en aspiration, et **en ce qu'**un robinet d'irrigation (23) est prévu pour fermer ou ouvrir la voie de raccordement (13) au canal d'irrigation (5), en amont de la voie de dérivation (27), respectivement un robinet d'aspiration (25) est prévu pour fermer ou ouvrir la voie de raccordement (15) au canal d'aspiration (9), en aval de la voie de dérivation (27).

3. Connecteur destiné à un système d'endoscopie selon la revendication 1 ou 2, comprenant une voie de communication (19,21) et un détecteur de pression (18a) pour détecter la pression dans cette voie de communication (19,21), **caractérisé en ce qu'**il comprend une voie borgne (29) et un deuxième détecteur de pression (18b) pour détecter la pression dans la voie borgne (29).

4. Connecteur selon la revendication 3, **caractérisé en ce qu'**il comprend une deuxième voie de communication (19,21).

5. Connecteur selon la revendication 4, **caractérisé en ce que** la voie de dérivation est disposée entre les deux voies de communication (19,21).

6. Connecteur selon la revendication 4, ou 5, **caractérisé en ce que** les détecteurs de pression (18a,18b) sont des détecteurs à membrane.

## Claims

1. An endoscopy system comprising a cannula (1) for housing an endoscope (3) and for forming, between the cannula and the endoscope, an irrigation channel (5) and outflow channel (9) respectively, intended for transporting an irrigation fluid and outflow fluid respectively, a coupling ring (11) mounted around the cannula (1) and provided with a coupling path (13, 15) for coupling to the irrigation channel (5), and to the outflow channel (9) respectively, and a connector (17) mounted on the coupling ring (11) and comprising a communication path (19, 21) for communicating with the coupling path (13, 15) and a first pressure sensor (18a) for sensing the pressure in the communication path (19, 21), **characterized in that** the coupling ring (11) is provided with a branch-off path (27) that communicates with the irrigation channel (5), and with the outflow channel (9) respectively, and **in that** the connector (17) includes a blind path (29) communicating with the branch-off path (27) and a second pressure sensor (18b) for sensing the pressure in this blind path (29).

2. The endoscopy system as claimed in claim 1, **characterized in that** the branch-off path (27) is placed downstream of the coupling path (13) for coupling to the irrigation channel (5) relative to the transport of the irrigating fluid and upstream of the coupling path (15) for coupling to the outflow channel (9) relative to the transport of the outflow fluid, and **in that** an irrigation tap (23) is provided for closing or opening the coupling path (13) to the irrigation channel (5), upstream of the branch-off path (27), and an outflow tap (25) is provided for closing or opening the coupling path (15) to the outflow channel (9), downstream of the branch-off path (27).

3. A connector intended for an endoscopy system as claimed in claim 1 or 2, comprising a communication path (19, 21) and a pressure sensor (18a) for sensing the pressure in this communication path (19, 21), **characterized in that** it includes a blind path (29) and a second pressure sensor (18b) for sensing the pressure in the blind path (29).

4. The connector as claimed in claim 3, **characterized in that** it includes a second communication path (19, 21).

5. The connector as claimed in claim 4, **characterized in that** the branch-off path is placed between the two communication paths (19, 21).

6. The connector as claimed in claim 4 or 5, **characterized in that** the pressure sensors (18a, 18b) are membrane sensors.

## Patentansprüche

1. Endoskopiesystem mit einer Kanüle (1) zur Aufnahme eines Endoskops (3) und zum Bilden eines Irrigationskanals (5) beziehungsweise eines Aspirationskanals (9) zwischen der Kanüle und dem Endoskop, der für die Beförderung eines Irrigationsfluids beziehungsweise Aspirationsfluids vorgesehen ist, mit einem Anschlussring (11), der um die Kanüle (1) herum angeordnet ist und einen Anschlusskanal (13, 15) zu dem Irrigationskanal (5) beziehungsweise zu dem Aspirationskanal (9) aufweist, und mit einem Verbindungsstück (17), das an dem Anschlussring (11) angebracht ist und über einen Verbindungskanal (19, 21) zu dem Anschlusskanal (13, 15) sowie über einen ersten Druckdetektor (18a) zum Detektieren eines Drucks in dem Verbindungskanal (19, 21) verfügt, **dadurch gekennzeichnet, dass** der Anschlussring (11) über einen Abzweigkanal (27) verfügt, der mit den Irrigationskanal (5) beziehungsweise mit dem Aspirationskanal (9) verbunden ist, und dass das Verbindungsstück (17) über einen blinden Kanal (29) verfügt, der mit dem Abzweigkanal (27) und mit einem zweiten Druckdetektor (18b) zum Detektieren eines Drucks in dem blinden Kanal (29) verbunden ist.

2. Endoskopiesystem nach Anspruch 1, **dadurch gekennzeichnet, dass** der Abzweigkanal (27) in Bezug auf den Transport des Fluids zur Irrigation strömungsabwärts von dem Anschlusskanal (13) zu dem Irrigationskanal (5) beziehungsweise in Bezug auf den Transport des Fluids zur Aspiration strömungsaufwärts von dem Anschlusskanal (15) zu dem Aspirationskanal (9) angeordnet ist und dass ein Irrigationshahn (23) zum Schließen oder Öffnen des Anschlusskanals (13) zu dem Irrigationskanal (5) strömungsaufwärts des Abzweigkanals (27) vorgesehen ist beziehungsweise ein Aspirationshahn (25) zum Schließen oder Öffnen des Anschlusskanals (15) zu dem Aspirationskanal (9) strömungsabwärts des Abzweigkanals (27) vorgesehen ist.

3. Verbindungsstück für ein Endoskopiesystem nach Anspruch 1 oder 2, das über einen Verbindungskanal (19, 21) und über einen Druckdetektor (18a) zum Detektieren des Drucks in dem Verbindungskanal (19, 21) verfügt, **dadurch gekennzeichnet, dass** es einen blinden Kanal (29) und einen zweiten Druckdetektor (18b) zum Detektieren des Drucks in dem blinden Kanal (29) aufweist.

4. Verbindungsstück nach Anspruch 3, **dadurch gekennzeichnet, dass** es über einen zweiten Verbindungskanal (19, 21) verfügt.

5. Verbindungsstück nach Anspruch 4, **dadurch gekennzeichnet, dass** der Abzweigkanal zwischen den beiden Verbindungskanälen (19, 21) angeordnet ist.

6. Verbindungsstück nach Anspruch 4 oder 5, **dadurch gekennzeichnet, dass** die Druckdetektoren (18a, 18b) Membrandetektoren sind.
